# EUROPEAN PATENT APPLICATION

(11) **EP 4 629 453 A1**
(43) Date of publication of application: **08.10.2025**
(21) Application number: 25167925.4
(22) Date of filing: 02.04.2025
(51) Int. Cl.: H01S 3/06, A61B 18/20, H01S 3/092, H01S 3/00, H01S 3/102, H01S 3/13, H01S 3/16

(54) **LASER DEVICE AND LASER TREATMENT APPARATUS**

(30) Priority: 05.04.2024 JP 2024061610
(71) Applicant: J. MORITA MANUFACTURING CORPORATION, Fushimi-ku, Kyoto-shi, Kyoto 6128533 (JP)
(72) Inventor: IWANE, Seiji, Fushimi-ku, Kyoto-shi, 612-8533 (JP)
(74) Representative: Müller Hoffmann & Partner

(57) **Abstract**

The presently disclosed laser device comprises: a resonator including a columnar solid-state laser rod, a first reflective film provided on a first end face of the solid-state laser rod, and a second reflective film provided on a second end face of the solid-state laser rod opposite to the first end face; and a flash lamp configured to excite the solid-state laser rod. The first reflective film and the second reflective film are of an ion beam sputtered coating film capable of preventing moisture from entering from outside air. The first reflective film is lower in reflectance than the second reflective film.

## Description

### CROSS REFERENCE TO RELATED APPLICATIONS

This application is based on Japanese Patent Application No. 2024-061610 filed on April 5, 2024 with the Japan Patent Office.

### BACKGROUND

### Field

The present disclosure relates to a laser device and a laser treatment apparatus. Description of the Background Art

In recent years, in the field of dentistry, laser light has been used for treatment such as dental caries and calculus removal, gingival incision, and the like. For example, for the treatment, a laser treatment apparatus is used that allows oscillation of an Er:YAG (erbium/yttrium aluminum garnet) laser having a wavelength of 2.94 µm. The laser treatment apparatus allowing oscillation of the Er:YAG laser incorporates a laser device comprising a resonator including a solid-state laser rod.

Such a resonator including a solid-state laser rod has a total reflection mirror and an output mirror disposed at opposite ends of the solid-state laser rod. Note, however, that infrared light having a wavelength of 2 µm or more is significantly absorbed with respect to an O-H group contained in water or the like, and when moisture in the air adsorbs to the total reflection mirror or the output mirror of the resonator, the adsorbed moisture absorbs the infrared light and generates heat, which damages the total reflection mirror or the output mirror and may deteriorate the resonator in performance in some cases.

Japanese Patent Laying-Open No. 08-316552 discloses a laser device comprising a solid-state laser rod having opposite ends provided with a beam tube in order to prevent moisture from adsorbing to a total reflection mirror or an output mirror of a resonator. The beam tube is filled with a liquid, such as fluorine-based oil, having a property of transmitting infrared light, and has a structure which prevents the resonator's total reflection and output mirrors from being exposed in the air.

### SUMMARY

However, the beam tube provided at the opposite ends of the solid-state laser rod needs to adopt a structure to seal a liquid, and it is also difficult to manage the sealed liquid, resulting in high cost for manufacturing and maintenance. In addition, the laser device disclosed in Japanese Patent Laying-Open No. 08-316552 will have a risk of a failure, that is, the liquid sealing structure is broken and the sealed liquid leaks.

The present disclosure has been made to solve the above-described problem, and an object of the present disclosure is to provide a laser device and a laser treatment apparatus having a resonator with a low risk of failure with respect to a mirror without deteriorated performance.

This is achieved by the claimed subject-matter.

The presently disclosed laser device is a laser device configured to emit laser light having a wavelength for which water has an absorption coefficient of 20 cm⁻¹ or more, and comprises: a resonator including a columnar solid-state laser rod, a first reflective film provided on a first end face of the solid-state laser rod, and a second reflective film provided on a second end face of the solid-state laser rod opposite to the first end face; and a flash lamp configured to excite the solid-state laser rod. The first and second reflective films are of an ion beam sputtered coating film capable of preventing moisture from entering from outside air. The first reflective film is lower in reflectance than the second reflective film.

The presently disclosed laser treatment apparatus is a laser treatment apparatus configured to treat an affected area using laser light and comprises the above-described laser device configured to emit laser light.

The foregoing and other objects, features, aspects and advantages of the present disclosure will become apparent from the following detailed description of the present disclosure when taken in conjunction with the accompanying drawings.

### BRIEF DESCRIPTION OF THE DRAWINGS

Fig. 1 shows an appearance of a laser treatment apparatus.
Fig. 2 shows an appearance of the laser treatment apparatus.
Fig. 3 is a diagram for illustrating a structure of a laser device.
Fig. 4 shows an appearance of a solid-state laser rod.

### DETAILED DESCRIPTION

Hereinafter, the present disclosure will be described with reference to the drawings. In the drawings, identical or equivalent components are identically denoted and will not be described repeatedly.

### [Configuration of Laser Treatment Apparatus]

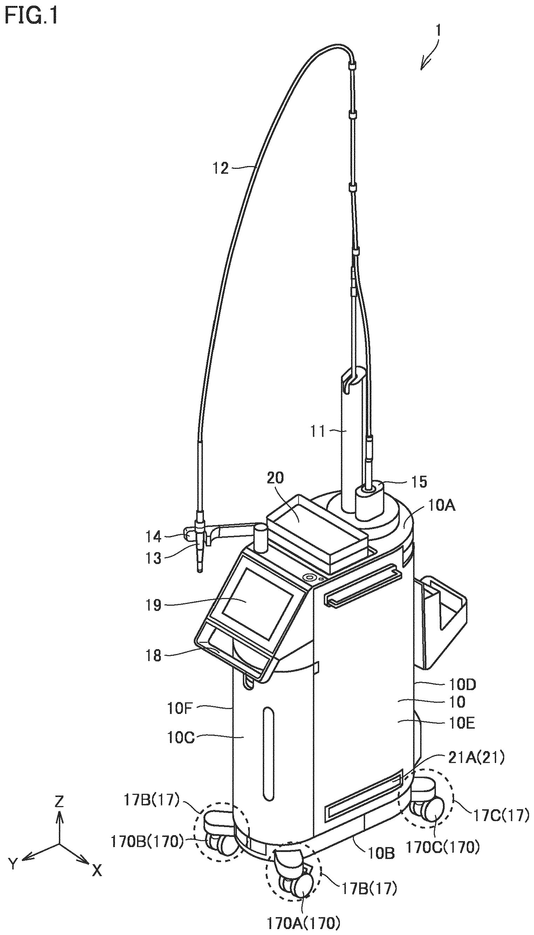

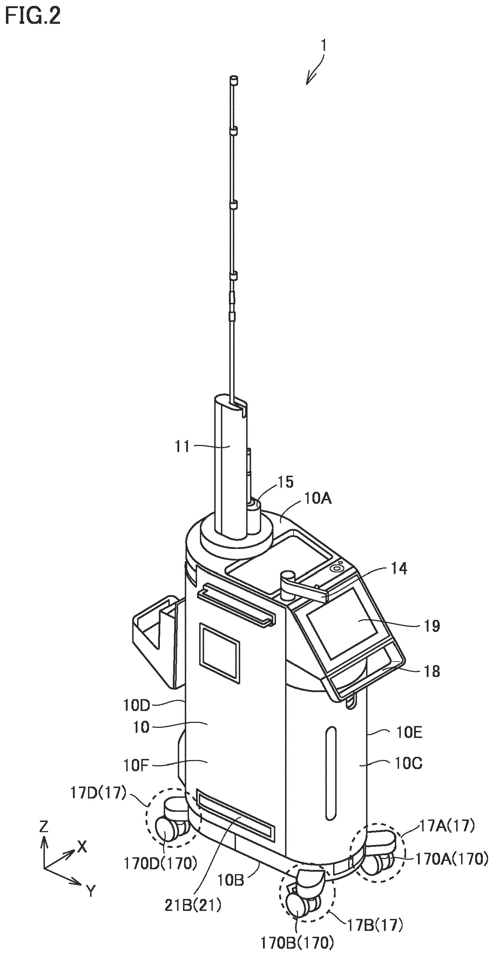

A main configuration of a laser treatment apparatus 1 will be described with reference to Figs. 1 and 2. Laser treatment apparatus 1 is used, for example, in dental diagnosis and treatment to treat teeth in the oral cavity of a patient. Laser treatment apparatus 1 is also applicable to fields other than dentistry, such as orthopedic surgery, otolaryngology, surgery, urology, dermatology, and ophthalmology.

Figs. 1 and 2 show an appearance of laser treatment apparatus 1. As shown in Figs. 1 and 2, laser treatment apparatus 1 comprises a housing 10. Housing 10 is formed in a rectangular parallelepiped or a substantially rectangular parallelepiped including a top surface 10A, a bottom surface 10B, a front surface 10C, a back surface 10D, a right side surface 10E, and a left side surface 10F, and accommodates a variety of types of configurations that laser treatment apparatus 1 comprises. Hereinafter, laser treatment apparatus 1 will be described while laser treatment apparatus 1 is installed on a surface for installation with an X axis representing an axis along a lateral direction of housing 10 (a direction of a shorter side of front surface 10C and back surface 10D), a Y axis representing an axis along a longitudinal direction of housing 10 (a direction of a shorter side of right side surface 10E and left side surface 10F), and a Z axis representing an axis along a direction of a height of housing 10 (a direction of a longer side of front surface 10C, back surface 10D, right side surface 10E, and left side surface 10F).

Laser treatment apparatus 1 comprises a connection unit 11, a waveguide 12, a handpiece 13, a holding unit 14, a connection unit 15, at least one leg unit 17, a handle 18, a display 19, and a tray 20.

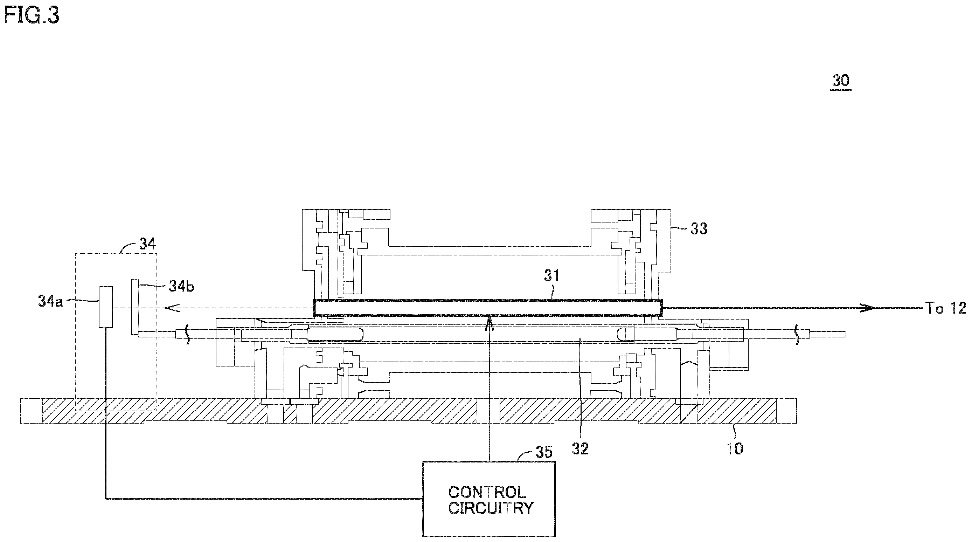

Connection unit 11 is provided on top surface 10A of housing 10 and has a pole along which waveguide 12 is extended. Allowing flexibly shaped waveguide 12 to extend along the pole, connection unit 11 enables a user to move waveguide 12 to a desired position. Connection unit 11 is configured to change as waveguide 12 is moved. Waveguide 12 extends flexibly and receives laser light transmitted from a laser device (see Fig. 3) provided inside housing 10 and transmits the received laser light to handpiece 13. Handpiece 13 receives the laser light transmitted through waveguide 12 and externally emits the received laser light.

Holding unit 14 is provided on top surface 10A of housing 10 rotatably on top surface 10A in an X-Y plane. Holding unit 14 has a distal end to hold handpiece 13 and thus secure handpiece 13 to housing 10.

Connection unit 15 is provided on top surface 10A of housing 10 and connects waveguide 12 to top surface 10A. Connection unit 15 interconnects the laser device provided inside housing 10 and waveguide 12, and interconnects a water passage (not shown) provided along waveguide 12 and a tube pump (not shown) provided inside housing 10. The water passage receives cleaning water supplied from the tube pump provided inside housing 10 and supplies the received cleaning water to handpiece 13. Handpiece 13 receives the cleaning water supplied through the water passage and externally ejects the received cleaning water.

At least one leg unit 17 is provided on bottom surface 10B of housing 10 and creates a gap between bottom surface 10B and the surface on which housing 10 is disposed for installation. Specifically, at least one leg unit 17 each includes at least one wheel 170. Housing 10 is in contact with the surface for installation via at least one wheel 170 provided at bottom surface 10B, and a gap is formed by at least one wheel 170 between bottom surface 10B and the surface for installation.

The laser treatment apparatus 1 is provided with four leg units 17A, 17B, 17C and 17D on bottom surface 10B of housing 10. The four leg units 17A, 17B, 17C and 17D include four wheels 170A, 170B, 170C and 170D, respectively. Hereinafter, the four leg units 17A, 17B, 17C and 17D are also collectively, simply referred to as "leg unit 17". The four wheels 170A, 170B, 170C and 170D are also collectively, simply referred to as "wheel 170". As wheel 170 rotates on the surface on which laser treatment apparatus 1 is installed, laser treatment apparatus 1 can move on the surface. Thus, for example, when a user uses laser treatment apparatus 1, the user can move laser treatment apparatus 1 to a place where a patient is present, and when the user does not use laser treatment apparatus 1, the user can move laser treatment apparatus 1 to a place for storage. Note that at least one leg unit 17 may not include at least one wheel 170 and instead simply fix housing 10 to the surface for installation.

Handle 18 is a component to be gripped by a user when the user moves laser treatment apparatus 1. Display 19 displays a variety of types of information for treatment of a patient using laser treatment apparatus 1. On tray 20 are disposed treatment instruments and the like necessary for a user to treat a patient using laser treatment apparatus 1.

Although Figs. 1 and 2 do not show where it is mounted, laser treatment apparatus 1 comprises a laser device (see Fig. 3) including a resonator (or an oscillator) configured to generate laser light. The resonator includes a solid-state laser rod and a flash lamp (or a light source), as will described hereinafter. The solid-state laser rod is, for example, a YAG crystal with Er added thereto as an active element. The solid-state laser rod is excited as it is irradiated with excitation light emitted from the flash lamp, and the resonator amplifies spontaneously emitted light to emit laser light.

The solid-state laser rod included in the oscillator is not limited to the YAG crystal with Er added thereto as an active element, or an Er:YAG rod, and may for example be a YAG crystal with any one of Er and Ho added thereto as an active element. Furthermore, the solid-state laser rod is not limited to a YAG crystal, and may be a solid-state laser medium doped with a lanthanoid rare earth element. The solid-state laser rod may for example be an Er;Cr:YSGG crystal, a Ho:YAG crystal, or the like.

Laser treatment apparatus 1 configured as described above allows a user holding handpiece 13 to extend waveguide 12 to dispose the distal end of handpiece 13 close to an affected area of a patient and thus expose the affected area to the laser light emitted from the distal end of handpiece 13 and the cleaning water ejected from the distal end of the handpiece. Thus, the user can treat the affected area using the laser light emitted by laser treatment apparatus 1.

### [Configuration of Laser Device]

Fig. 3 is a diagram for illustrating a structure of laser device 30. Laser device 30 comprises a resonator having a columnar solid-state laser rod 31 and a flash lamp 32 configured to excite solid-state laser rod 31. Laser device 30 further comprises a support member 33 configured to hold and fix solid-state laser rod 31 to housing 10 of laser treatment apparatus 1, a detection device 34 configured to detect laser light, and control circuitry 35 configured to control an output of flash lamp 32 based on the detected laser light.

Laser treatment apparatus 1 used in a medical field such as dental treatment allows laser light applied to an affected area to be absorbed by major components of a living tissue of the affected area, that is, water and hydroxyapatite, to perform treatment by incision, hemostasis, coagulation, and vaporization. For the treatment, it is necessary to use laser light having a high absorptance locally for the affected area to minimize an effect of transmitted light on surrounding healthy tissues. Accordingly, a laser light having a wavelength for which water has a high absorption coefficient (of 20 cm⁻¹ or more) is preferable, which is different from a wavelength generally used in industrial applications.

For example, when laser device 30 employs a Ho:YAG crystal for solid-state laser rod 31, laser device 30 emits laser light for which water has an absorption coefficient of 32 cm⁻¹. Furthermore, when laser device 30 employs an Er:YAG crystal and an Er;Cr:YSGG crystal for solid-state laser rod 31, laser device 30 emits laser light for which water has absorption coefficients of 12000 cm⁻¹ and 5000 cm⁻¹, respectively (D. J. Segelstein, "The complex refractive index of water," University of Missouri-Kansas City (1981)). Furthermore, for laser treatment apparatus 1 used in a medical field such as dental diagnosis and treatment, it is preferable that laser device 30 emit pulsed laser light in order to reduce a thermal effect on surrounding healthy tissues during incision and vaporization. Specifically, laser device 30 preferably emits pulsed laser light having a pulse width of 10 µs to 1000 µs.

Solid-state laser rod 31 is excited as it is irradiated with excitation light emitted from flash lamp 32, and it needs to constitute a resonator in order to amplify spontaneously emitted light. For a general laser device, a resonator is composed of an output mirror (a partial reflection mirror) disposed at a position separated by a predetermined distance from one end of a solid-state laser rod, and a total reflection mirror disposed at a position separated by a predetermined distance from the other end of the solid-state laser rod.

However, infrared light having a wavelength for which water has an absorption coefficient of 20 cm⁻¹ or more is significantly absorbed with respect to an O-H group contained in water or the like, and when moisture in the air adsorbs to the total reflection mirror or the output mirror of the resonator, the adsorbed moisture absorbs the infrared light and generates heat, and may thus damage the total reflection mirror or the output mirror.

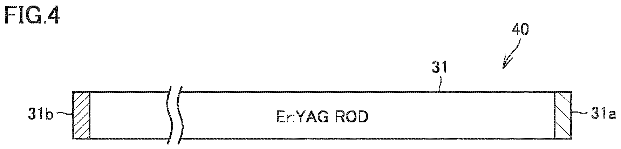

Accordingly, the present laser device 30 does not have a configuration provided with a total reflection mirror and an output mirror separately from solid-state laser rod 31; rather, it adopts a configuration in which a reflective film formed of an ion beam sputtered coating film capable of preventing moisture from entering from outside air is directly formed on an end face of solid-state laser rod 31. Fig. 4 shows an appearance of solid-state laser rod 31.

As shown in Fig. 4, solid-state laser rod 31 can have one end face (or a first end face) provided with a first reflective film 31a and has the other end face (or a second end face) provided with a second reflective film 31b to configure a resonator 40 to cause laser oscillation. That is, first and second reflective films 31a and 31b directly coat the end faces of solid-state laser rod 31 so that a reflection plane constituting a mirror of resonator 40 is not in contact with outside air. Thus, laser device 30 does not need to be provided with a total reflection mirror or an output mirror, and resonator 40 having no risk of failure relevant to the mirrors without poor performance can be implemented.

First and second reflective films 31a and 31b are of an ion beam sputtered coating film. The ion beam sputtered coating film is a film having high density and can prevent moisture from permeating to a reflection plane of first and second reflective films 31a and 31b. Insofar as first and second reflective films 31a and 31b are formed of a film having high density, they are not limited to the ion beam sputtered coating film and may be deposited in a different method.

Specifically, first and second reflective films 31a and 31b are made of Al₂O₃, Ta₂O₅, or SiO₂. Furthermore, it is preferable that first and second reflective films 31a and 31b be of a film of multiple layers having at least one layer including a film made of Ta₂O₅. As a matter of course, insofar as first and second reflective films 31a and 31b can ensure necessary reflectance, they are not limited to Al₂O₃, Ta₂O₅, or SiO₂.

First reflective film 31a corresponds to an output mirror, and can reflect light emitted from a laser crystal excited by excitation light emitted from flash lamp 32 and emit amplified light as laser light. Second reflective film 31b corresponds to a total reflection mirror and reflects light emitted from the laser crystal excited by the excitation light emitted from flash lamp 32. Accordingly, first reflective film 31a is lower in reflectance than second reflective film 31b.

For example, first reflective film 31a has a reflectance of about 92%, and second reflective film 31b has a reflectance of about 99.4%. While second reflective film 31b may have a reflectance of 100% to function as a total reflection mirror, the laser device 30 uses laser light passing through second reflective film 31b to monitor and control the laser light emitted from the end face provided with first reflective film 31a.

Specifically, laser device 30 detects the laser light that passes through second reflective film 31b by detection device 34. Detection device 34 includes a detector 34a configured to detect the laser light that passes through second reflective film 31b, and an optical filter 34b provided between detector 34a and the end face provided thereon with second reflective film 31b, and configured to shield visible light. Detector 34a is, for example, a pyroelectric element made of lithium tantalate (LiTaO₃)/lead zirconate titanate (PZT). Detector 34a is not limited to the above configuration insofar as it can detect infrared light having a wavelength of 2 µm or more.

Optical filter 34b is a visible light cutting filter made for example of silicon (Si)/germanium (Ge). Optical filter 34b can filter out stray light other than the laser light passing through second reflective film 31b (the excitation light from flash lamp 32, in particular), and detector 34a can accurately detect the laser light passing through second reflective film 31b. As a matter of course, optical filter 34b may be dispensed with insofar as a countermeasure against stray light is not required for detector 34a.

Based on intensity of laser light detected by detector 34a, control circuitry 35 monitors intensity of laser light emitted from the end face provided with first reflective film 31a. Furthermore, based on the intensity of the laser light detected by detector 34a, control circuitry 35 can also control the output of flash lamp 32 to control the intensity of the laser light emitted from the end face provided with first reflective film 31a to a set value.

Although not shown, control circuitry 35 includes a processor and a memory. The processor executes a variety of types of programs stored in the memory to control the intensity of the laser light emitted from the end face provided with first reflective film 31a based on a set value received at an input unit.

The processor is configured by a CPU, a GPU, or the like, and can read and execute a program (for example, an OS and a control program) stored in the memory. The processor executes a variety of types of programs read from the memory. The memory includes, for example, a nonvolatile storage device such as a ROM or a flash memory. The memory stores a control program in addition to an OS for implementing a basic function.

The input unit is not limited to any specific device, and may for example be a touch panel superimposed on display 19. A function provided by the processor executing a program may partially or entirely be implemented using a dedicated hardware circuit (e.g., an ASIC, an FPGA, or the like).

One method for monitoring the laser light emitted from the end face provided with first reflective film 31a is a method for spectrally dispersing a portion of the laser light emitted from the end face provided with first reflective film 31a, and detecting the spectrally dispersed laser light. This method requires a spectroscopic optical system, which increases the device in size. In addition, the spectroscopic optical system spectrally disperses the laser light emitted from the end face provided with first reflective film 31a, and requires an optical element having large endurance against laser light intensity.

The laser device 30 has second reflective film 31b that functions as a total reflection mirror to be reduced in reflectance slightly from 100% to transmit laser light and has detection device 34 to have a function to detect the transmitted laser light, and is thus reduced in size. As a matter of course, laser device 30 may be configured to be provided with a spectroscopic optical system to spectrally disperse a portion of the laser light emitted from the end face provided with first reflective film 31a.

Furthermore, laser device 30 including solid-state laser rod 31 having end faces provided with first and second reflective films 31a and 31b can dispense with a total reflection mirror and an output mirror provided as separate components and can be reduced in size as well as cost for the components. Furthermore, providing first and second reflective films 31a and 31b on the end faces of solid-state laser rod 31 parallelized with high precision can reduce a cost for a precise mechanical component configuration for obtaining parallelism between the total reflection mirror and the output mirror as well as a work for adjustment.

Furthermore, first and second reflective films 31a and 31b provided on the end faces of solid-state laser rod 31 that are formed of an ion beam sputtered coating film having high density can significantly reduce a risk of damage caused by adsorption of moisture as done to a total reflection mirror and an output mirror. Furthermore, as solid-state laser rod 31 having end faces provided with first and second reflective films 31a and 31b constitutes resonator 40, a problem such as dust entering resonator 40 does not occur, and laser device 30 is enhanced in reliability.

It should be understood that the examples disclosed herein are illustrative and non-restrictive in any respect. The present disclosure is defined by the claimed subject-matter, and is intended to encompass any modification that falls within the claimed subject-matter.

## Claims

1. A laser device configured to emit laser light having a wavelength for which water has an absorption coefficient of 20 cm⁻¹ or more, comprising:
a resonator including a columnar solid-state laser rod, a first reflective film provided on a first end face of the solid-state laser rod, and a second reflective film provided on a second end face of the solid-state laser rod opposite to the first end face; and
a flash lamp configured to excite the solid-state laser rod,
the first reflective film and the second reflective film being of an ion beam sputtered coating film capable of preventing moisture from entering from outside air,
the first reflective film being lower in reflectance than the second reflective film.

2. The laser device according to claim 1, wherein the solid-state laser rod is a YAG crystal or a YSGG crystal with at least one of Er, Ho, and Cr added thereto as an active element.

3. The laser device according to claim 1 or 2, wherein the laser light emitted by the laser device is pulsed laser light having a pulse width of 10 µs to 1000 µs.

4. The laser device according to any one of claims 1 to 3, wherein the first reflective film and the second reflective film are made of Al₂O₃, Ta₂O₅, or SiO₂.

5. The laser device according to any one of claims 1 to 3, wherein the first reflective film and the second reflective film are of a film of multiple layers having at least one layer including a film made of Ta₂O₅.

6. The laser device according to any one of claims 1 to 5, further comprising a detector configured to detect laser light passing through the second reflective film.

7. The laser device according to claim 6, further comprising an optical filter provided between the detector and the second end face provided with the second reflective film, and configured to shield visible light.

8. The laser device according to claim 7, wherein the optical filter is made of silicon or germanium.

9. The laser device according to any one of claims 6 to 8, further comprising control circuitry configured to control an output of the flash lamp based on the laser light detected by the detector.

10. A laser treatment apparatus configured to treat an affected area with laser light, comprising
a laser device according to any one of claims 1 to 9 and configured to emit laser light.
